Europäisches Patentamt

European Patent Office

Office européen des brevets

(19)

(11) Numéro de publication : **0 115 973 B1**

(12) **FASCICULE DE BREVET EUROPÉEN**

(45) Date de publication du fascicule du brevet :
07.09.88

(51) Int. Cl.⁴ : **A 61 M   1/00**

(21) Numéro de dépôt : **84400017.4**

(22) Date de dépôt : **05.01.84**

(54) **Dispositif de traitement de l'hydrocéphalie avec filtre à impédance variable.**

(30) Priorité : **14.01.83 FR 8300546**

(43) Date de publication de la demande :
**15.08.84 Bulletin 84/33**

(45) Mention de la délivrance du brevet :
**07.09.88 Bulletin 88/36**

(84) Etats contractants désignés :
**AT BE CH DE FR GB IT LI LU NL SE**

(56) Documents cités :
**EP-A- 0 068 815
US-A- 3 769 982
US-A- 3 889 687
US-A- 3 894 541**

(73) Titulaire : **UNIVERSITE RENE DESCARTES, (PARIS V)**
**12 rue de l'Ecole de Médecine**
**F-75006 Paris (FR)**

(72) Inventeur : **Sainte Rose, Christian**
**14 rue Corbon**
**F-75015 Paris (FR)**
Inventeur : **Lacombe, Jacques**
**17 rue Mathis Appart. A 25**
**F-75019 Paris (FR)**

(74) Mandataire : **Martin, Jean-Jacques et al**
**Cabinet REGIMBEAU 26, Avenue Kléber**
**F-75116 Paris (FR)**

Jouve, 18, rue St-Denis, 75001 Paris, France

## Description

L'invention concerne le traitement de l'hydrocéphalie.

Les dispositifs de traitement de l'hydrocéphalie couramment utilisés comprennent un cathéter intracrânien connecté à une tubulure, munie d'une valve, et permettant la dérivation de l'écoulement du liquide céphalo-rachidien vers une ou plusieurs cavités de l'organisme capables d'absorber ce liquide. La cavité est en règle générale le péritoine ou l'oreillette droite du coeur.

Le principe des valves utilisées depuis 20 ans repose sur une pression intracrânienne maintenue à un niveau constant quel que soit le débit. On a ainsi utilisé divers types de valves anti-retour à seuil de pression de déclenchement, et parfois des clapets anti-siphon.

Ces dispositifs ne donnent pas entière satisfaction : ils aboutissent à un écrètement de toutes les variations de la pression intracrânienne, qu'elles soient normales (lors des efforts, lors du sommeil paradoxal) ou non ; par ailleurs, ils maîtrisent mal les effets d'aspiration dues aux brusques dépressions provoquées dans la tubulure lors du passage de la position couchée à la position assise ou de celle-ci à la position debout.

De tels types de valves ont été décrits notamment dans les brevets américains US-A-3 889 687 et US-A-3 769 982. Si ces dispositifs permettent un drainage du liquide céphalo-rachidien en excès, ils présentent l'inconvénient d'un fonctionnement par tout ou rien.

La présente invention vient proposer un dispositif qui va à l'encontre du principe de maintien de la pression intracrânienne à un niveau constant.

A cet effet, selon l'invention, la tubulure du dispositif de traitement est munie, en série sur la valve, d'un filtre hydraulique à impédance variable non-linéaire, autorisant les variations rapides de la pression intracrânienne, qui possèdent un temps caractéristique de l'ordre de la seconde, tout en empêchant une variation lente de cette pression, avec un temps caractéristique de l'ordre de la minute, par le maintien d'un débit moyen de liquide céphalo-rachidien, voisin du débit physiologique, ledit filtre hydraulique à impédance variable non linéaire étant constitué par au moins une bille et au moins un orifice d'écoulement du liquide céphalo-rachidien formant un siège de soupape à débit de fuite variable avec la pression.

Dans un mode de réalisation préférentiel de l'invention, le filtre hydraulique est placé sur le trajet vertical de la tubulure ; la ou les billes sont logées, mobiles, sur le passage du liquide céphalo-rachidien, entre un orifice d'entrée et un orifice de sortie de diamètres inférieurs à celui de la ou des billes ; et l'orifice de sortie forme avec la ou les billes un siège de soupape à débit de fuite, définissant le débit moyen précité.

Typiquement, le débit de fluide est de l'ordre de 8 à 30 cm³ par heure pour une différence de pression statique de 5 à 40 cm d'eau aux bornes du filtre.

Un tel filtre hydraulique peut avantageusement être incorporé à une valve anti-retour à seuil de pression de déclenchement fixe.

Dans un premier mode de réalisation particulier, le filtre comprend une seule bille, l'orifice de sortie étant surfacé pour former avec cette bille ledit siège de soupape à débit de fuite. Le diamètre de la bille est d'environ 2 mm.

Dans un autre mode de réalisation particulier, le filtre comporte un grand nombre de microbilles, les orifices d'entrée et de sortie étant formés par des filtres munis de perforations de largeur inférieure au diamètre des microbilles. De préférence, les microbilles possèdent un diamètre compris entre 0,1 et 0,6 mm. En pratique, elles occupent entre 50 et 90 % du volume interne de la chambre qui les loge, entre les orifices d'entrée et de sortie.

Le filtre de la présente invention se combine bien avec un cathéter intracrânien qui comporte, à distance prédéterminée de son extrémité libre, munie d'orifices destinés à communiquer avec les ventricules cérébraux, une protubérance de positionnement, et, au voisinage de celle-ci côté extrémité libre, un ou plusieurs orifices intermédiaires destinés à communiquer avec les espaces sous-arachnoïdiens de la cavité crânienne, ce qui permet d'éviter l'inversion du gradient de pression entre les cavités ventriculaires et la périphérie du parenchyme cérébral.

D'autres caractéristiques et avantages de l'invention apparaîtront à l'examen de la description détaillée ci-après et des dessins annexés, sur lesquels :

— les figures 1A et 1B illustrent très schématiquement le traitement de l'hydrocéphalie par dérivation vers le péritoine et vers l'oreillette droite du coeur, respectivement ;

— La figure 2 représente un premier mode de réalisation de filtre selon l'invention ;

— la figure 3 en représente une variante dans laquelle le filtre est incorporé à une valve ;

— la figure 4 représente un second mode de réalisation de filtre selon l'invention ;

— la figure 5 en représente une variante dans laquelle le filtre est incorporé à une valve ; et

— la figure 6 représente un cathéter perfectionné utilisé préférentiellement dans le dispositif de la présente invention.

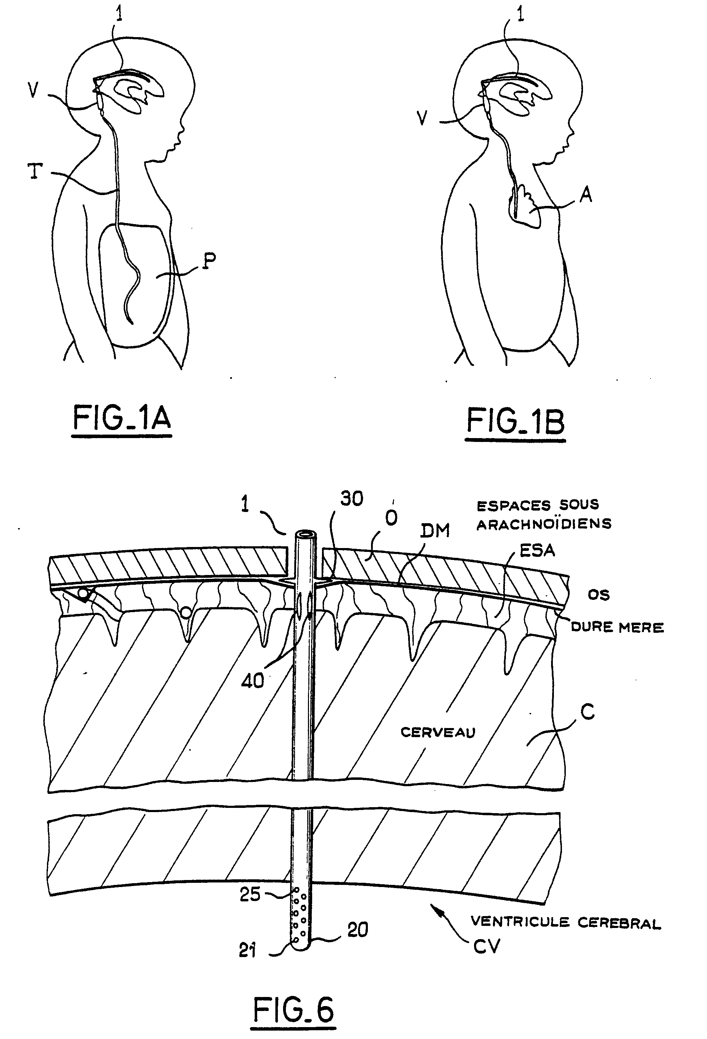

Sur la figure 1A, on voit un cathéter intracrânien 1 relié par une tubulure T comprenant une valve V à la cavité péritonéale P d'un enfant hydrocéphale. La figure 1B représente un montage analogue, mais vers l'oreillette droite du coeur A.

Ces dispositifs de traitement de l'hydrocéphalie sont pratiquement les seuls retenus à l'heure actuelle, après des tentatives dans de très nombreuses directions. Ils posent cependant encore des problèmes, dont le plus grave est la complication par hématome sous-dural. La cause en est, la plupart du temps, un drainage excessif du liquide céphalo-rachidien depuis les ventricules cérébraux. Les valves anti-retour à seuil de pression de déclenchement, même associées à un clapet anti-siphon, ne peuvent empêcher complètement ce phénomène.

Par ailleurs, ces valves ont pour effet de maintenir la pression intracrânienne à un niveau constant, quel que soit le débit de liquide céphalo-rachidien. Mais cela écrête toutes les variations, même normales, de la pression intracrânienne. On a observé en effet les variations normales suivantes de cette pression :

— en permanence, variations rapides de faible amplitude liées au pouls et à la respiration ;

— pendant le sommeil paradoxal, s'y superposent des variations plus lentes, et de grande amplitude, plutôt erratiques ;

— pendant l'éveil, se superposent aux variations rapides précitées des variations également plus lentes, d'amplitude moyenne, assez régulières.

Les interactions qui sont la cause de ces variations de pression sont mal connues : on ne sait pas encore expliquer complètement les variations de la pression intracrânienne observées pendant le sommeil paradoxal. En revanche, leur écrêtement par les valves à seuil de pression n'est pas conforme à la réalité physiologique.

Dans ces conditions, la présente invention a pour but de fournir un dispositif de traitement de l'hydrocéphalie qui fonctionne de manière plus proche de la physiologie normale.

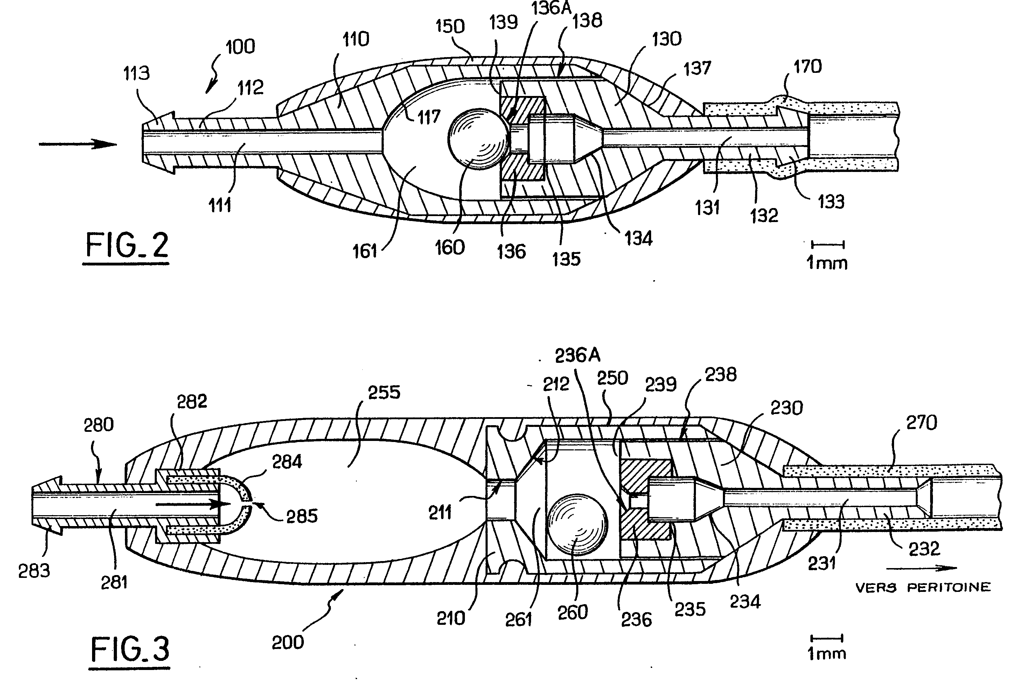

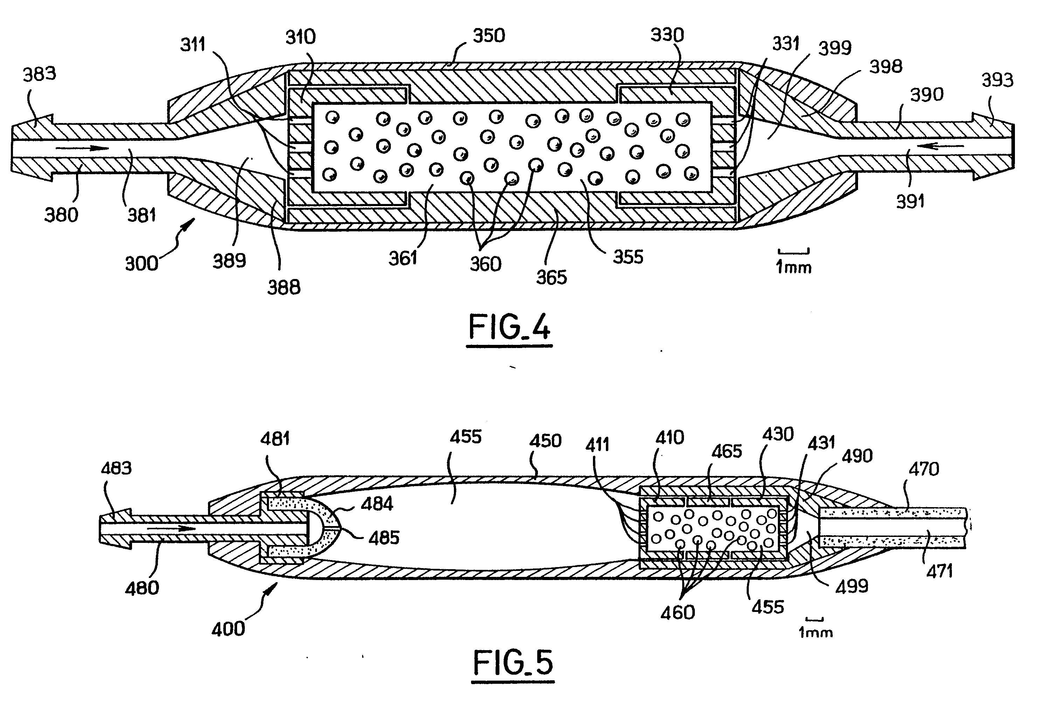

A cet effet, un filtre spécial (figure 2 ou figure 4) est inséré sur le trajet vertical de la tubulure T, en amont ou en aval de la valve V. Le filtre peut aussi être purement et simplement incorporé à la valve (figure 3 ou figure 5, respectivement).

On décrira tout d'abord en référence aux figures 2 et 3 le premier mode de réalisation de l'invention, dans lequel le filtre comporte une seule bille.

Le filtre de la figure 2 est désigné généralement par la référence 100. Il comporte deux corps principaux 110 et 130, qui sont collés l'un sur l'autre autour d'une surface de révolution 138. A son extrémité gauche, le corps 110 est percé d'un canal 111, qui aboutit dans une protubérance 112 formant embout de raccordement, avec un épaulement 113. Intérieurement, du côté du corps 130, le corps 110 définit à partir du canal 111 une surface de raccordement 117 formant paraboloïde vers la surface cylindrique 138. De son côté, le corps 130 est muni d'un tube d'extrémité 132 définissant un canal de sortie 131. Le tube 132 est également muni d'un épaulement 133 permettant la fixation de la tubulure 170. La pièce 130 comporte un raccordement conique 137 entre sa tubulure 132 et la surface cylindrique 138 déjà citée. Intérieurement, le canal 131 se continue vers la gauche par une section conique 134, puis une section cylindrique 135, qui loge un siège de soupape 136 pour une bille 160 logée dans la cavité 161. Cette cavité est définie entre la surface 117 déjà citée et la partie en section droite 139 de la pièce 130.

A l'intérieur de la cavité 161 est défini un orifice d'entrée constitué par le débouché du canal 111. L'orifice de sortie est défini par la partie 136. Ces deux orifices possèdent un diamètre inférieur à celui de la bille 160. D'un autre côté, l'orifice 136A forme avec la bille 160 un siège de soupape 136 à débit de fuite choisi, ce débit de fuite demeurant bien entendu variable avec la pression. Une manière de réaliser cela consiste à définir l'orifice 136A de telle façon qu'il soit complètement obturé par la bille 160, puis à en découper une partie supplémentaire, sur un arc couvrant une partie de la circonférence, pour assurer le débit de fuite précité. L'ensemble du filtre est entouré par une gaine 150 en matériau mou.

Le siège de soupape 136 peut être réalisé en matériau mou. On augmente ainsi la non-linéarité du filtre : plus la pression augmente, plus le débit de fluide diminue.

Dans un mode de réalisation particulier, la bille 160 possède un diamètre d'environ 2 mm, et est réalisée en un matériau bio-implantable, de préférence métallique, tel que de l'acier inoxydable. On prendra soin que la coopération entre la bille 160 et le siège 136 s'effectue sur une surface limitée, de manière à éviter un collage de la bille en position d'obturation sur le siège de soupape du fait des tensions superficielles. De leur côté, les pièces 110 et 130 sont réalisées en un matériau bio-implantable rigide, tel qu'un polycarbonate ou un métal du genre acier inoxydable. La gaine 150 est réalisée en un matériau bio-implantable élastique tel que du silicone. De même, le tube 170 est réalisé de préférence en silicone. D'autres indications de forme peuvent être tirées de la figure 2, qui est représentée à l'échelle, de même que les figures 3 à 5.

La forme du siège de soupape 136 définie par la surface par rapport la bille 160 est ajustée afin d'assurer un débit de fuite d'environ 20 cm$^3$ par heure sous une pression de 40 cm d'eau, lorsque la bille est en position de fermeture.

Le fonctionnement est le suivant :

— en cas de variations importantes du débit sur un sujet couché, la bille 160 est chassée vers le siège 136, et contrecarre cette augmentation brutale du débit.

— en cas de changement de position (position couchée vers position assise en particulier), un début d'effet de siphon entraîne aussitôt une mise en place de la bille sur son siège 136, ce qui augmente la résistance à l'écoulement et réduit le siphonnage. La bille demeure le plus souvent en place pendant que le sujet est assis ou debout.

On comprendra mieux le fonctionnement du dispositif selon la présente invention en faisant maintenant référence à la figure 3, qui illustre le filtre dans une réalisation où il est incorporé à une valve à seuil de pression.

Sur la figure 3, on retrouve la pièce 230, qui correspond à la pièce 130 de la figure 2, les références numériques étant simplement augmentées de 100 unités. On notera que le tube 270 est fixé sans épaulement sur l'embout 232. La forme de la pièce 210, qui correspond à la pièce 110 de la figure 2, a un peu varié : sa surface intérieure 212 est conique, et son orifice 211 est un peu plus grand, tout en demeurant inférieur au diamètre extérieur de la bille 260. L'enveloppe 250 définit en amont du filtre une chambre 255, dans laquelle aboutit l'embout proximal 280. Celui-ci définit intérieurement un canal 281, et est muni extérieurement d'un épaulement 283 pour la fixation d'une tubulure. L'embout 280 est également muni d'une lèvre extérieure 282 qui forme une pince pour tenir une valve 284 équipée d'une fente 285. De manière connue, on réalise ainsi une valve à seuil de pression de déclenchement. La pression amont est liée à la pression dans les ventricules cérébraux. Lorsqu'elle atteint une valeur suffisante, elle ouvre la fente 285, ce qui permet le passage du liquide céphalo-rachidien dans la cavité 255 et par conséquent vers le filtre situé après celle-ci.

On retrouve le fonctionnement déjà décrit à propos de la figure 2, étant observé que l'écoulement de liquide céphalo-rachidien ne peut se produire que si la différence de pression au niveau de la valve 284 avec sa fente 285 est suffisante pour ouvrir celle-ci.

On voit immédiatement que les dispositions de la présente invention vont à l'encontre du principe couramment admis jusqu'à présent, et consistant comme précédemment indiqué à maintenir la pression intracrânienne à niveau constant quel que soit le débit chez le sujet couché.

Pour éviter le siphonnage en position assise ou debout, il avait été proposé d'utiliser des clapets anti-siphon.

La présente invention préconise l'addition à une valve d'un filtre hydraulique passe-bas non-linéaire, présentant en pratique deux états.

Chez le sujet couché, une augmentation lente, anormale, de la pression intracrânienne sera contrôlée par le filtre, qui peut alors laisser passer un débit important (hyper sécrétion de liquide céphalo-rachidien, par exemple). A l'inverse, les variations rapides de la pression intracrânienne s'exerceront normalement (sommeil paradoxal, cri, etc.).

En position assise ou debout, la bille se place la plupart du temps dans son logement. Cela augmente l'impédance du filtre, et évite le siphonnage du liquide céphalo-rachidien, et l'abaissement de la pression intracrânienne en dehors des limites physiologiques.

En pratique, il a été observé que ce débit de fuite varie de 8 à 25 cm³ par heure, lorsque la différence de pression statique aux bornes du filtre augmente de 5 à 50 cm d'eau.

On décrira maintenant en référence aux figures 4 et 5 un second mode de réalisation de l'invention, dont le filtre utilise une multitude de microbilles.

La figure 4 illustre un filtre seul, et destiné à être placé en série, en amont ou en aval, avec une valve à seuil de pression de déclenchement. Ce filtre est défini entre un embout proximal 380 et un embout distal 390. L'embout proximal 380 est muni d'un épaulement pour la fixation d'une tubulure, notée 383. Il comporte intérieurement un canal 381 qui s'évase en 389 au niveau d'un évasement correspondant 388 de l'embout. De même, l'embout distal 390 définit intérieurement un canal 391, dont l'amont s'évase en 399 au niveau d'un élargissement 398 de l'embout. Le logement du filtre est défini par une pièce généralement cylindrique 365 montée entre les deux embouts. L'ensemble des deux embouts et de cette pièce est entouré par une gaine 350. Intérieurement, la pièce 365 comporte à ses deux extrémités des décrochements logeant respectivement deux pièces collées 310 et 330, formant des filtres munis de perforations axiales 311 et 331 respectivement. Dans un mode de réalisation préférentiel, les perforations 311 et 331 sont des fentes dont la largeur est inférieure au diamètre des microbilles 360 qui sont logées dans la cavité interne 355 du filtre. On notera que les microbilles ne sont pas à l'échelle et qu'elles sont illustrées en nombre limité pour simplifier le dessin.

Il s'est avéré possible de choisir le diamètre et le nombre des microbilles 360, le volume de la chambre 355, et la taille des perforations des filtres 310 et 330 pour obtenir les caractéristiques suivantes :

— en position verticale, les billes étant agglomérées vers le filtre distal 330, on a un débit de fuite de 8 à 25 cm³ par heure pour une différence de pression statique aux bornes du filtre allant de 5 à 50 cm d'eau.

— en position horizontale, les billes n'occupent qu'une partie du volume interne de la chambre, ne formant donc pas obstacle à l'écoulement du liquide céphalo-rachidien. Typiquement, les microbilles occupent entre 50 et 90 % du volume interne de la chambre qui les loge, ce qui laisse largement passer le liquide céphalo-rachidien lorsque cette chambre est en position horizontale. Dans un mode de réalisation particulier, les microbilles possèdent un diamètre compris entre 0,1 et 0,6 mm.

Le fonctionnement général est sensiblement le même que précédemment :

— en cas de variations importantes du débit, notamment chez un sujet couché, les microbilles sont chassées vers le filtre distal 330 et en s'agglomérant elles contrecarrent l'augmentation brutale du débit. Cela permet de maintenir en grande partie les variations de pression qui accompagnent les phases d'éveil et de sommeil paradoxal.

— en cas de changement de position, en particulier au passage de la position couchée vers la position assise, un début d'effet de siphon entraîne aussitôt une agglomération des billes vers le filtre distal 330, augmentant la résistance à l'écoulement et réduisant le siphonnage. Les billes demeurent généralement agglomérées tant que le sujet est assis ou debout.

Pour le reste, toute augmentation lente et anormale de la pression intracrânienne est contrariée par le maintien d'un débit moyen de liquide céphalo-rachidien au niveau du filtre selon la présente invention.

La figure 5 illustre, à échelle plus réduite, un filtre semblable à celui de la figure 4, mais incorporant également la valve à seuil de pression de déclenchement. Cette valve apparaît dans la partie gauche de la figure 5, et est très proche de celle de la figure 3. Les références numériques des éléments qui se correspondent commencent par 4 au lieu de 2. Cette partie ne sera donc pas décrite à nouveau. En partie droite, on retrouve les éléments principaux du filtre de la figure 4, à savoir une enveloppe extérieure 490 qui loge deux filtres respectivement proximaux et distaux 410 et 430, collés à l'intérieur de l'enveloppe, avec interposition d'une entretoise 465. Comme précédemment, les filtres 410 et 430 sont munis de perforations 411 et 431 constituées avantageusement de fentes. A l'intérieur de la chambre 455 définie par ce filtre est logée une multitude de microbilles 460, dont le diamètre est supérieur à la largeur des perforations 411 et 431. Les autres caractéristiques du filtre sont déterminées comme précédemment indiqué.

Dans un mode de réalisation particulier, applicable aux figures 4 et 5, les microbilles sont en verre siliconé. Les pièces à hachures descendantes de la gauche vers la droite sont réalisées en matière plastique bio-implantable dure, telle que du polycarbonate. Les pièces hachurées dans l'autre sens sont réalisées en un matériau bio-implantable élastique, tel que du silicone. Les pièces matérialisées en coupe par des pointillés sont des tubes de silicone. Il en est de même pour les valves à seuil de pression de déclenchement illustrées en 284 et 484.

Bien que le dispositif de la présente invention, tel que décrit en référence aux figures 2 à 5, donne déjà en lui même d'excellents résultats, il se combine avantageusement avec l'usage d'un cathéter particulier, illustré sur la figure 6.

Cette figure représente en O l'os crânien, qui surmonte la dure-mère DM, puis l'arachnoïde, et les espaces sous-arachnoïdiens ESA. Par des villosités, ceux-ci soutiennent la masse C du cerveau, dont l'intérieur délimite des ventricules cérébraux CV. Un cathéter normal 1 se contente de pénétrer par une ouverture de l'os O pour aller jusqu'aux ventricules cérébraux, où son extrémité proximale 20 est munie de perforations 21 à 25 permettant l'évacuation du liquide céphalo-rachidien. Le cathéter perfectionné est muni d'un épaulement 30, qui vient prendre appui entre l'os et la dure-mère, et est immédiatement suivi d'orifices intermédiaires 40. Ceux-ci permettent également d'extraire le liquide baignant les espaces sous-arachnoïdiens, afin d'éviter une inversion du gradient des pressions entre les deux côtés de la masse du cerveau C. La différence de calibre global entre les trous d'extrémités 21 à 25 et les orifices intermédiaires 40 doit être suffisante pour que l'action de pompage du liquide céphalo-rachidien s'exerce d'abord sur les espaces sous-arachnoïdiens, et ensuite seulement sur les ventricules cérébraux. D'autres détails descriptifs concernant ce cathéter sont contenus dans la demande de brevet français Nº 8 300 545 FR-A-2 539 298 déposée ce jour au nom de la demanderesse, et intitulée « Dispositif pour le traitement d'hydrocéphalie avec cathéter perfectionné ».

Il a été précédemment indiqué que le filtre de l'invention permet notamment une réduction des effets de siphonnage lors des changements de position du sujet. Le cathéter décrit ci-dessus se combine avantageusement avec ce filtre pour éviter encore l'apparition d'une différence de pression néfaste entre les deux faces de la masse cervicale C. Le risque d'hématome sous-dural se trouve alors encore amoindri.

Bien entendu, la présente invention n'est pas limitée aux modes de réalisation décrits, mais s'étend à toutes variantes incluses dans le cadre des revendications ci-après.

**Revendications**

1. Dispositif de traitement de l'hydrocéphalie, comprenant un cathéter intracrânien (1) connecté à une tubulure (T), munie d'une valve (V), et permettant la dérivation de l'écoulement du liquide céphalo-rachidien vers une ou plusieurs cavités (P, A) de l'organisme capables d'absorber ce liquide, caractérisé par le fait que ladite tubulure (T) est munie, en série sur la valve (V), d'un filtre hydraulique à impédance variable non-linéaire (100, 200, 300, 400), autorisant les variations rapides de la pression intracrânienne, qui possèdent un temps caractéristique de l'ordre de la seconde, tout en empêchant une variation lente de pression, avec un temps caractéristique de l'ordre de la minute, par le maintien d'un débit moyen de liquide céphalo-rachidien, voisin du débit physiologique, ledit filtre hydraulique à impédance variable non-linéaire (100, 200, 300, 400) étant constitué par au moins une bille (160, 260, 360, 460) et au moins un orifice d'écoulement (136A, 236A) du liquide céphalo-rachidien formant un siège de soupape (136, 236) à débit de fuite variable avec la pression.

2. Dispositif selon la revendication 1, caractérisé par le fait que le filtre hydraulique (100, 200, 300, 400) est placé sur le trajet vertical de la tubulure (T), que la ou les billes (160, 260, 360, 460) sont logées, mobiles, sur le passage du liquide céphalo-rachidien, entre un orifice d'entrée (161, 261, 311, 411) et un orifice de sortie (136A, 236A, 331, 431) de diamètres inférieurs à celui de la ou des billes, et que l'orifice de sortie forme avec la ou les billes un siège de soupape à débit de fuite définissant le débit moyen précité.

3. Dispositif selon la revendication 2, caractérisé par le fait que le débit de fuite est de l'ordre de 8 à 30 cm$^3$ par heure pour une différence de pression statique de 5 à 40 cm d'eau aux bornes du filtre.

4. Dispositif selon l'une des revendications 2 ou 3, caractérisé par le fait que le filtre hydraulique (200, 400) est incorporé à une valve anti-retour à seuil de pression de déclenchement fixe.

5. Dispositif selon l'une des revendications 2 à 4, caractérisé par le fait que le filtre (100, 200) comporte une seule bille (160, 260), l'orifice de sortie étant surfacé pour former avec cette bille ledit siège de soupape à débit de fuite.

6. Dispositif selon la revendication 5, caractérisé par le fait que le diamètre de la bille est d'environ 2 mm.

7. Dispositif selon l'une des revendications 2 à 4, caractérisé par le fait que le filtre (300, 400) comporte un grand nombre de microbilles (360, 460), les orifices d'entrée et de sortie étant formés par des filtres (310, 330 ; 410, 430) munis de perforations de largeur inférieure au diamètre des microbilles.

8. Dispositif selon la revendication 7, caractérisé par le fait que les microbilles possèdent un diamètre compris entre 0,1 et 0,6 mm.

9. Dispositif selon l'une des revendications 7 ou 8, caractérisé par le fait que les microbilles occupent entre 50 et 90 % du volume interne de la chambre qui les loge entre les orifices d'entrée et de sortie.

10. Dispositif selon l'une des revendications 1 à 9, caractérisé par le fait que le cathéter intracrânien (1) comporte, à distance prédéterminée de son extrémité libre (20), munie d'orifices (21-25) destinés à communiquer avec les ventricules cérébraux (CV), une protubérance de positionnement (30), et, au voisinage de celle-ci côté extrémité libre, un ou plusieurs orifices intermédiaires (40) destinés à communiquer avec les espaces sous-arachnoïdiens (ESA) de la cavité crânienne, ce qui permet d'éviter l'inversion du gradient de pression entre les cavités ventriculaires et la périphérie du parenchyme cérébral.

**Claims**

1. A hydrocephaly treatment device comprising an intracranial catheter (1) connected to a tube (T) fitted with a first valve (V) for diverting the flow of cephalo-rachidian liquid to one or more cavities (P, A) in the organism capable of absorbing said liquid, characterised in that a hydraulic filter (100, 200, 300, 400) of non-linear variable impedance is connected in said tube in series with said valve, thereby permitting the intracranial pressure to vary in the short term over a characteristic period of about one second, while preventing said pressure from varying in the long term over a characteristic period of about one minute, by maintaining an average flow rate of the cephalorachidian liquid close to the physiological flow rate said hydraulic filter of non-linear variable impedance (100, 200, 300, 400) comprising at least a single bead (160, 260, 360, 460) and an outlet orifice (136A, 236A) for the cephalo-rachidian liquid forming the seat (136, 236) of a leakage rate valve with a flow rate related to pressure.

2. A device according to claim 1, characterized in that said hydraulic filter (100, 200, 300, 400) is located in a vertical portion of the path of said tube (T), the at least one bead (160, 260, 360, 460) being movably retained in the path of the cephalo-rachidian liquid between an inlet orifice (161, 261, 311, 411) and an outlet orifice (136A, 236A, 331, 431), said orifices having a diameter smaller than the one of said at least one bead, and wherein said outlet orifice co-operates with said at least one bead to constitute a leakage flow rate valve defining said average flow rate.

3. A device according to claim 2, characterized in that said leaksge flow rate is about 8 cm³ to 30 cm³ per hour for a static pressure difference across said filter of 5 cm to 40 cm of water.

4. A device according to claim 2, characterized in that said hydraulic filter (200, 400) is incorporated in a common structure with said first valve, and wherein said first valve is a non-return valve which opens at a fixed threshold pressure.

5. A device according to claim 2, characterized in that said filter (100, 200) includes one bead only (160, 260), and wherein said outlet orifice constitutes a bead-receiving seat for said leakage flow rate valve.

6. A device according to claim 5, characterized in that the diameter of said bead is about 2 mm.

7. A device according to claim 2, characterized in that said filter (300, 400) includes a large number of microbeads (360, 460), said inlet and outlet orifices being in the form of respective filters (310, 330 ; 410, 430) having perforations of width smaller than the diameter of said microbeads.

8. A device according to claim 7, characterized in that said microbeads are of a diameter lying in the range 0.1 mm to 0.6 mm.

9. A device according to claim 7 or 8, characterized in that said microbeads are housed in a chamber between said filter inlet and outlet orifices, and wherein said microbeads occupy 50 % to 90 % of the internal volume of said chamber.

10. A device according to any preceding claim 1 to 9, characterized in that said intracranial catheter (1) has a free (20) end fitted with orifices (21-25) for communication with the cerebral ventricles (CV), and wherein said catheter further includes a positioning member (30) located a predetermined distance from said free end together with one or more intermediate orifices (40) located adjacent to said stop member and on the free end side thereof, said intermediate orifices being for communication with the sub-arachnoid spaces (ESA) of the cranial cavity, thereby making it possible to avoid inverting the pressure gradient between the ventricular cavities and the periphery of the cerebral parenchyma.

**Patentansprüche**

1. Vorrichtung zur Behandlung des Hydrozephalus, mit einem interkranialen Katheter (1), das an eine mit einem Ventil (V) versehene und das Ableiten des Liquor cerebrospinalis-Flusses in eine oder mehrere Körperhöhlen, die diesen Liquor absorbieren können, ermöglichende Röhre (T) angeschlossen ist, dadurch gekennzeichnet, daß die Röhre (T) in Reihe mit dem Ventil (V) mit einem hydraulischen Filter variabler, nicht linearer Impedanz (100, 200, 300, 400) versehen ist, um durch Aufrechterhalten einer mittleren Abflußmenge an Liquor cerebrospinalis nahe der physiologischen, Abflußmenge schnelle Änderungen des interkranialen Druckes, die eine charakteristische Zeit in Sekunden-Größenordnung haben, zuzulassen und eine Langsame Druckänderung mit einer charakteristischen Zeit in Minuten-Größenordnung zu verhindern, wobei dieser hydraulische Filter mit variabler, nicht-linearer Impedanz (100, 200, 300, 400) aus wenigstens einer Kugel (160, 260, 360, 460) und wenigstens einer Abflußöffnung (136A, 236A) für Liquor cerebrospinalis besteht, welche einen Ventilsitz (136, 236) für die sich mit dem Druck ändernde Abflußmenge bildet.

2. Vorrichtung nach Anspruch 1, dadurch gekennzeichnet, daß der hydraulische Filter (100, 200, 300, 400) im vertikalen Bereich der Röhre (T) vorgesehen ist, daß die Kugel oder die Kugeln (160, 260, 360, 460) vom durchfließenden Liquor cerebrospinalis beweglich zwischen einer Eintrittsöffnung (161, 261, 311, 411) und einer Austrittsöffnung (136a, 236a, 331, 431), deren Durchmesser jeweils geringer ist als der der Kugel oder der Kugeln, angeordnet sind, und daß die Austrittsöffnung zusammen mit der Kugel oder den Kugeln einen Ventilsitz für die die oben genannte mittlere Abflußmenge definierende Abflußmenge bildet.

3. Vorrichtung nach Anspruch 2, dadurch gekennzeichnet, daß die Abflußmenge für eine statische Druckdifferenz von 5 bis 40 cm Wassersäule an den Grenzen des Filters in der Größenordnung von 8 bis 30 cm³ pro Stunde liegt.

4. Vorrichtung nach Anspruch 2 oder 3, dadurch gekennzeichnet, daß der hydraulische Filter (200, 400) in ein Rückschlagventil mit festem Druckschwellenwert eingegliedert ist.

5. Vorrichtung nach einem der Ansprüche 2 bis 4, dadurch gekennzeichnet, daß der Filter (100, 200) eine einzige Kugel (160, 260) aufweist, wobei die Austrittsöffnung so gestaltet ist, daß sie mit der Kugel den Ventilsitz für die Abflußmenge bildet.

6. Vorrichtung nach Anspruch 5, dadurch gekennzeichnet, daß der Durchmesser der Kugel ungefähr 2 mm ist.

7. Vorrichtung nach einem der Ansprüche 2 bis 4, dadurch gekennzeichnet, daß der Filter (300, 400) eine große Anzahl von Mikrokugeln (360, 460) aufweist, wobei die Eintrittsöffnungen und die Austrittsöffnungen durch Filter (310, 330 ; 410, 430) gebildet sind, die mit Löchern kleiner als der Durchmesser der Mikrokugeln versehen sind.

8. Vorrichtung nach Anspruch 7, dadurch gekennzeichnet, daß die Mikrokugeln einen Durchmesser zwischen einschließlich 0,1 und 0,6 mm aufweisen.

9. Vorrichtung nach Anspruch 7 oder 8, dadurch gekennzeichnet, daß die Mikrokugeln zwischen 50 und 90 Prozent des inneren Volumens der Kammer ausfüllen, in der sie zwischen den Eintritts- und den Austrittsöffnungen angeordnet sind.

10. Vorrichtung nach einem der Ansprüche 1 bis 9, dadurch gekennzeichnet, daß der interkraniale Katheter (1) in vorbestimmter Entfernung zu seinem freien Ende (20), das mit öffnungen (21-25) versehen ist, die mit den Hirnventrikeln (CV) kommunizieren sollen, einen Vorsprung (30) zum Positionieren aufweist und, benachbart dazu in Richtung des freien Endes, eine oder mehrere Zwischen-öffnungen (40) enthält, die mit den subarachnoidalen Räumen der Schädelhöhle kommunizieren sollen, um zu vermeiden, daß der Druckgradient zwischen den Höhlenöffnungen und der Peripherie des Hirnparenchyms sich umkehrt.

FIG_1A

FIG_1B

FIG_6

FIG. 2

FIG. 3

VERS PERITOINE

0 115 973

FIG.4

FIG.5